# EUROPEAN PATENT SPECIFICATION

(11) **EP 1 270 599 B1**
(45) Date of publication and mention of the grant of the patent: **31.03.2004**
(21) Application number: 00981747.9
(22) Date of filing: 15.12.2000
(51) Int. Cl.: C08B 37/08, A61K 31/726, C08B 37/00, A61K 31/715

(54) **SALMON-ORIGIN CHONDROITIN SULFATE**
CHONDROITINSULFAT AUS LACHSEN
SULFATE DE CHONDROITINE ISOLE A PARTIR DE SAUMON

(30) Priority: 02.03.2000 JP 2000057688
(43) Date of publication of application: 02.01.2003
(73) Proprietor: Takai, Mitsuo, Sapporo-shi, Hokkaido 064-0917 (JP)
(72) Inventor: Takai, Mitsuo, Sapporo-shi, Hokkaido 064-0917 (JP); Kono, Hiroyuki, Sapporo-shi, Hokkaido 007-0828 (JP)
(74) Representative: Cresswell, Thomas Anthony
(86) International application number: PCT/JP2000/008894
(87) International publication number: WO 2001/064756

(56) References cited:
- JP-A- 9 291 011
- JP-B- 43 000 082
- US-A- 5 888 984
- TADAAKI TAKEDA ET AL.: 'Sake hana nankotsu yurai chondroitin ryusan no gulcose chokan kyushu ni oyobosu eikyo' NIHON EIYOU SHOKURYOU GAKKAISHI vol. 51, no. 4, 1998, pages 213 - 217, XP002936703

## Description

### Technical Field

The present invention relates to a novel chondroitin sulfate which is expected to be useful for various purposes in the fields of drugs, cosmetics, food additives.

### Background Art

A chondroitin sulfate is an acid mucopolysaccharide contained in a connective tissue of animals. This is made of a disaccharide recurring structure of D-glucuronic acid and a sulfated N-acetylgalactosamine, and there are various isomers depending on sulfation of a hydroxyl group of a constituent sugar.

Sites to be sulfated are hydroxyl groups in the 2- and 3-positions of glucuronic acid, a hydroxyl group in the 2-position of iduronic acid and hydroxyl groups in the 4- and 6-positions of N-acetylgalactosamine. A chondroitin sulfate chain is a linear polysaccharide having a molecular weight of 10⁴ to 10⁵, and present as a proteoglycan covalently bound to a core protein. Generally, as a chondroitin sulfate chain occurring in nature, one made only of a recurring unit of one type of a sulfated disaccharide rarely exists, and it usually contains various kinds of sulfated or non-sulfated disaccharides at different ratios.

A chondroitin sulfate is a substance which was found earliest among acid mucopolysaccharides. In 1886, it was separated from cartilage by Fischer and Boedeker, and first named chondroit acid. After that, it was found to have a sulfate ester, and called chondroitin sulfate. Further, in 1951, it was found by Meyer et al. that a chondroitin sulfate includes three types (A, B and C) and A and C are present in cartilage.

As a proteoglycan having a chondroitin sulfate chain, there are aggrecan, versican, decholin and the like. However, their functions are unknown in many cases. Nevertheless, since an activity of aglycan to control a cartilage tissue function or an anti-cell-adhesion activity of versican is completely lost by chondrokinase treatment, a chondroitin sulfate chain is considered to have such activities. Further, a chondroitin sulfate proteoglycan was isolated as a neurotrophic factor of retinal neurons and an axon elongation factor. These activities were also lost by chondrokinase treatment. Lately, there is also a report that a disaccharide derived from a chondroitin sulfate suppresses activation of natural killer cells. Moreover, it was known before the advent of human immunodefficiency virus (HIV) that sulfated polysaccharides such as chondroitin sulfates, carrageenans, dextran sulfates and the like inhibit infection of cells with a large number of viruses such as influenza virus, herpes simplex virus and the like.

Since the chondroitin sulfate is thus found to have various physiological activities and properties, it has been used in drugs such as an anti-inflammatory agent and the like, further in cosmetics or eye lotion as a humectant and in food additives' such as a gelling agent, a jellying agent and the like. In dairy life, it can be found in an unexpectedly wide range. Moreover, in addition to these ordinary applications, a latent use value is expected as drugs for various purposes from its characteristics.

The chondroitin sulfate currently used for medical care or the like includes chondroitin sulfate A (ChS-A, chondroitin 4-sulfate) extracted from whale cartilage and chondroitin sulfate C (ChS-C, chondroitin 6-sulfate) extracted froma shark fin. Recently, because of whaling ban, weight has been being shifted from ChS-A to ChS-C. Nevertheless, a price of a shark fin is increasing as a food material of Chinese food. For this reason, a raw material and a method by which the chondroitin sulfate can be obtained in a large amount at lower cost have been in demand. Takeda et al. (J. JPN. Soc Nutr. Food Sci., 51, 213-217) disclosed the preparation of chondroition sulfate and its oligosaccharide mixture from salmon nasal cartilage and investigated the effects on intestinal absorption of glucose.

Accordingly, the invention aims to provide a novel chondroitin sulfate which can be obtained in a large amount at lower cost and expected to be useful for various purposes, and a method for obtaining the same.

### Disclosure of the Invention

The invention relates to a chondroitin sulfate comprising a disaccharide unit containing a non-sulfated N-acetyl-D-galactosamine (hereinafter abbreviated as non-sulfated GalNAc): (11.0 ± 3.3)%, a disaccharide unit containing a C6-monosulfated N-acetyl-D-galactosamine (hereinafter abbreviated as C6-monosulfated GalNAc): (52.8 ± 15.8)%, a disaccharide unit containing a C4-sulfated N-acetyl-D-galactosamine (hereinafter abbreviated as C4-monosulfated GalNAc): (28.4 ± 8.5)% and a disaccharide unit containing a C4,C6-disulfated N-acetyl-D-galactosamine (hereinafter abbreviated as C4,C6-disulfated GalNAc): (7.8 ± 2.3)%, a process for producing the chondroitin sulfate, which comprises grinding salmon nasal cartilage at a low temperature, defatting the ground matter, then treating it with an alkali and a pronase, centrifuging the resulting liquid digested matter, and then precipitating it with ethanol, and the process for producing the chondroitin sulfate, which further comprises treating the resulting precipitate with a cation exchange resin. Moreover, the invention relates to an anti-inflammatory agent and a humectant containing the chondroitin sulfate.

### Best Mode for Carrying Out the Invention

The chondroitin sulfate of the invention is obtained in a large amount from, for example, salmon nasal cartilage.

The outline of the process is as described below.

That is, first, a product obtained by removing the skin, the hard bone, the meat particles from the head discharged during processing of a salmon, separating only nasal cartilage and grinding it at -120°C is used as a raw material. The grinding at the low temperature can prevent degradation and oxidation of the product owing to heat generation in grinding. Further, in the grinding at normal temperature, fine grinding to an impossible size is enabled, and a particle size is also rendered uniform. An acid mucopolysaccharide is extracted from the cartilage powder, and this is treated with a proton exchange resin to obtain a chondroitin sulfate fraction. The operating method can be based on a method for extracting a chondroitin sulfate from whale or shark cartilage.

The production of a purified chondroitin sulfate from the cartilage powder is described in more detail, and the outline is as mentioned below.

That is, the cartilage powder is first defatted with an organic solvent such as acetone. Then, this is treated with an alkaline aqueous solution such as a sodium hydroxide aqueous solution, neutralized, and then digested with a pronase such as actinase E. Subsequently, the liquid digested matter is centrifuged, and pH is changed to acidity with acetic acid. Ethanol is then added to form a precipitate. The resulting precipitate is collected through centrifugal separation, and washed with ethanol, and this is then vacuum-dried.

The thus-obtained acid mucopolysaccharide is dissolved in a small amount of deionized water, and this is treated with - a cation exchange resin such as DOWEX 50WX2. The elute is then neutralized, and this is dialyzed against deionized water. The thus-obtained solution is concentrated, filtered such as through a membrane filter, and then freeze-dried to obtain a purified product of the chondroitin sulfate.

An existing ratio of disaccharides constituting the chondroitin sulfate of the invention is usually
non-sulfated GalNAc (%): 11.0 ± 3.3
C6-monosulfated GalNAc (%): 52.8 ± 15.8
C4-monosulfated GalNAc (%): 28.4 ± 8.5
C4,C6-disulfated GalNAc (%): 7.8 ± 2.3, preferably
non-sulfated GalNAc (%): 11.0 ± 2.2
C6-monosulfated GalNAc (%): 52.8 ± 10.6
C4-monosulfated GalNAc (%): 28.4 ± 5.7
C4,C6-disulfated GalNAc (%): 7.8 ± 1.6.

Such an existing ratio of disaccharides constituting the chondroitin sulfate has been unknown so far.

In a whale cartilage-origin chondroitin sulfate known so far, C4-monosulfated GalNAc is as large as approximately 70%, while C4,C6-disulfated GalNAc is as small as 1% or less. Also in a shark cartilage-origin chondroitin sulfate known so far, C6-monosulfated GalNAc is as large as approximately 70%, while C4-monosulfated GalNAc is as small as 10-odd %.

Further, with respect to a distribution of a sulfuric acid group, the chondroitin sulfate of the invention has a more random structure than the ordinary ones. That is, it has a structure that a disaccharide unit containing non-sulfated GalNAc, a disaccharide unit containing C6-monosulfated GalNAc, a disaccharide unit containing C4-monosulfated GalNAc and a disaccharide unit containing C4,C6-disulfated GalNAc are randomly arranged.

The chondroitin sulfate of the invention, like the ordinary ones, can be used in drugs such as an anti-inflammatory agent and the like, further in cosmetics or eye lotion as a humectant and in food additives such as a gelling agent, a jellying agent.

Moreover, since the chondroitin sulfate has a high physiological viscosity, an effect of prolonging a local residence time of combined drugs by blending with the drugs is considered for the future development. In addition, it has been reported that the chondroitin sulfate expedites stabilization of corneal collagen filaments and is effective for maintaining a function of an eye tissue. Accordingly, the application of it as a highly functional skin substitute by blending with collagens such as these extracted from the salmon skin, the cattle skin is also considered. Moreover, since it has not only physiological and pharmaceutical activities but also characteristics as a polymeric electrolyte, its industrial application is also possible. Since the chondroitin sulfate of the invention has an intermediate structure between the whale-origin chondroitin sulfate and the shark-origin chondroitin sulfate, it can be expected to find relatively wide acceptance.

### Examples

The invention is illustrated more specifically below by referring to Examples. However, the invention is not limited at all to these Examples.

### Example 1

### (1) Defatting of cartilage

A product obtained by removing the skin, the hard bone, the meat particles from the head discharged during processing of a salmon, separating only nasal cartilage and grinding it at -120°C under liquid nitrogen was used as a raw material.
1) Approximately 100 mg of the cartilage powder was charged into a 2,000-milliliter conical flask, and 700 ml of acetone was added. The mixture was stirred for 10 minutes.
2) The reaction mixture was allowed to stand for 5 minutes, and a supernatant solution was removed.
3) The procedure of 1)-2) was further repeated three times.
4) The remaining precipitate was dried with a vacuum desiccator.
5) The resulting sample was stored at -30°C.

### (2) Alkali treatment

1) Five grams of the defatted cartilage powder was dissolved in 80 ml of 0.2 M NaOH.
2) The mixture was stirred in a water bath of 37°C for 3 hours.
3) The reaction mixture was neutralized to pH of 7.0 with acetic acid.

### (3) Pronase digestion

1) Ten milliliters of a 0.2 M Tris-HCl buffer solution (pH 7.8) was added.
2) calcium acetate was added to a final concentration of 0.02 M.
3) For preservation, 5 ml of methanol was added.
4) Fifty milligrams of actinase E was added.
5) The mixture was slowly stirred in a water bath of 37°C for 48 hours.

### (4) Ethanol precipitation

1) The liquid digested matter was centrifuged at 10,000 rpm and 4°C for 30 minutes.
2) The supernatant was suction-filtered using a 0.45-micrometer membrane filter.
3) Calcium acetate equivalent to 5% was added to the filtrate.
4) The mixture was adjusted to pH of 4.5 with acetic acid.
5) A 2-fold amount of ethanol was added, and the mixture was allowed to stand for 48 hours.

### (5) Washing and drying of a precipitate

1) The ethanol solution was centrifuged at 7,000 rpm and 4°C for 30 minutes.
2) The precipitate was recovered, and 300 ml of 80% ethanol was added. The solution was slowly stirred for 12 hours.
3) The solution was centrifuged at 10,000 rpm and 4°C for 30 minutes.
4) The procedure of 2)-3) was repeated.
5) To the precipitate, 200 ml of 100% ethanol was added, and the mixture was slowly stirred for 6 hours.
6) The reaction mixture was centrifuged at 10,000 rpm and 4°C for 30 minutes.
7) The thus-obtained precipitate (acid mucopolysaccharide) was dried with a vacuum desiccator. (Apparent yield from the defatted cartilage powder: 44.0%)

### (6) Purification of chondroitin sulfate

### (6-1) Pretreatment

1) One hundred milliliters of a cation exchange resin DOWEX 50WX2 was stirred in 3 N HCl for 2 hours, washed with water, and then stirred in 2 N NaOH for 2 hours.
2) The foregoing procedure was repeated three times, and the reaction mixture was then washed with water.
3) An absorbent cotton was packed under a column of 2.5 x 40 cm, and a resin was charged so as to prevent passage of air.

### (6-2) Treatment with a cation exchange resin DOWEX 50Wx2

1) The acid mucopolysaccharide obtained in (5) above was dissolved in quite a small amount of deionized water.
2) 1) was passed through a column, and allowed to stand for 20 minutes.
3) Through the column, 400 ml (approximately 4 times the volume of the resin) of deionized water was passed.
4) The elute was immediately neutralized with 1N NaOH.

### (6-3) Purification

1) The neutralized solution was dialyzed against deionized water for 3 days.
2) The resulting solution was concentrated to 20 ml with an evaporator.
3) The product was filtered with a 0.22-meter membrane filter, and then freeze-dried to obtain a dry sample. (Apparent yield from the defatted cartilage powder: 24.0%)

### Results of analysis

The results and the considerations of the analysis on the above-obtained chondroitin sulfate (hereinafter abbreviated as ChS-S) of the invention are described below. By the way, in the composition analysis, the structural analysis and the like thereof, the determinations of amino sugar and uronic acid were conducted by the Morgan-Elson method and the Bitter-Muir method. Further, the degree of substitution of the sulfuric acid group was measured by the Rhodizonate method and the elemental analysis. In measuring the molecular weight and the purity of ChS-S, GPC, FT-IR and cellulose acetate electrophoresis were properly used. In the structural analysis, ¹³C-NMR (100 MHz) and ¹H-NMR (400 MHz) were used. Further, the determination of the position for substitution of sulfuric acid and its distribution were conducted by HPLC of an unsaturated disaccharide obtained by two-step lyase decomposition of ChS-S with chondroitinase ABC and chondroitinase ACII and two-dimensional NMR (COSY ¹H-NMR hydrogen nucleus shift correlation or the like) analysis. As control samples, commercial chondroitin 4-sulfate (ChS-A, whale cartilage origin) and chondroitin 6-sulfate (ChS-C, shark cartilage origin)(both made by Seikagaku Kogyo K.K.) were used.

### (1) Quantitative determination of uronic acid

The ratio of uronic acid contained in each sample solution and the chondroitin sulfate content (purity) obtained from this value were shown in Table 1, and the influence of treatment with the cation exchange resin (DOWEX 50WX2) (hereinafter abbreviated as proton exchange treatment) on purity and yield in Table 2 respectively. By the way, the uronic acid content of standard chondroitin sulfate is 37%.

From these results, it was found that the chondroitin sulfate having quite a high purity was obtained by this process. Further, the purity is much increased after the proton exchange treatment in comparison to that before the proton exchange treatment. From this fact, it is considered that other acid mucopolysaccharides such as hyaluronic acid, dermatan sulfate and the like can completely be removed by the proton exchange treatment.

**Table 1**

| Uronic acid content and purity | | | | |
|---|---|---|---|---|
| | ChS-S before purification | ChS-S | ChS-A | ChS-C |
| Uronic acid (%) | 25.50 | 36.49 | 35.63 | 36.88 |
| Purity (%) | 68.92 | 98.62 | 96.30 | 99.68 |

**Table 2**

| Yield of chondroitin sulfate of the invention in each step | | | |
|---|---|---|---|
| | Apparent yield (%) | Purity(%) | Yield (%) |
| Defatted cartilage | 100 | ... | ... |
| Ethanol precipitation | 44.0 | 68.9 | 30.3 |
| Proton exchange treatment | 24.0 | 98.6 | 23.7 |

### (2) Analysis of an amino sugar

The N-acetylgalactosamine (GalNAc) content of each sample solution was shown in Table 3. Further, although approximately 0.3% of N-acetylglucosamine was detected from the acid mucopolysaccharide before the proton exchange treatment, it was not identified at all in ChS-S, ChS-A and

ChS-C. None of other amino sugars was contained therein. From this fact, it could be identified that the amino sugar constituting the chondroitin sulfate was only N-acetylgalactosamine and other amino sugars could completely be removed by the proton exchange treatment.

**Table 3**

| N-acetylgalactosamine content of chondroitin sulfate | | | | |
|---|---|---|---|---|
| | ChS-S before purification | ChS-S | ChS-A | ChS-C |
| GalNAc (%) | 25.46 | 32.62 | 30.70 | 26.73 |

### (3) Elemental analysis

The weight ratio of carbon, hydrogen, nitrogen, oxygen and sulfur contained in each sample and the degree of sulfation were shown in Tables 4 and 5 respectively. From Table 4, it was identified that all of the samples showed the same composition. From Table 5, the results were obtained that the shark. cartilage-origin chondroitin sulfate had the highest degree of sulfation and the chondroitin sulfate of the invention had the slightly lower degree of sulfation in comparison to the others.

**Table 4**

| Results of the composition analysis of chondroitin sulfate | | | | | |
|---|---|---|---|---|---|
| | C (%) | H (%) | N (%) | O (%) | S (%) |
| ChS-S | 39.86 | 5.48 | 3.25 | 45.91 | 5.50 |
| ChS-A | 39.96 | 5.45 | 3.30 | 45.40 | 5.89 |
| ChS-C | 40.97 | 5.62 | 3.15 | 43.98 | 6.28 |

**Table 5**

| Content of a sulfuric acid group per molecule of chondroitin sulfate | | |
|---|---|---|
| ChS-S | ChS-A | ChS-C |
| 0.72 | 0.77 | 0.80 |

### (4) Quantitative determination of a sulfuric acid group

The sulfuric acid group contained in each sample and the ratio of sulfur calculated therefrom were shown in Table 6. From the results, it was found that ChS-C had the slightly higher content of the sulfuric acid group and ChS-S and ChS-A had approximately the same content thereof. Further, the results agreed with the results of the elemental analysis described in (3) above.

**Table 6**

| Contents of sulfuric acid and sulfur of each chondroitin sulfate | | |
|---|---|---|
| | Sulfuric acid group (%) | Sulfur (%) |
| ChS-S | 18.62 | 6.15 |
| ChS-A | 18.52 | 6.15 |
| ChS-C | 21.74 | 7.17 |

### (5) Measurement of a molecular weight

An average molecular weight and a molecular weight distribution of each sample were shown in Table 7. The average molecular weight of the chondroitin sulfate of the invention was 173,000. While this value is slightly higher than the others, the molecular weight distributions are almost the same. It is generally known that a molecular weight of a chondroitin sulfate amounts to 50,000 to 300,000 by an extraction method.

The molecular weight distribution of the chondroitin sulfate obtained this time also agreed therewith.

**Table 7**

| Average molecular weight and molecular weight distribution of chondroitin sulfate | | | |
|---|---|---|---|
| | ChS-S | ChS-A | ChS-C |
| Average molecular weight | 173,000 | 151,000 | 108,000 |
| Molecular Weight distribution | 5-30 x 10⁴ | 5-30 x 10⁴ | 5-30 x 10⁴ |

### (6) HPLC

The existing ratio of disaccharides constituting each chondroitin sulfate obtained by fractionating the chondroitin sulfate subjected to enzymolysis using a combination of two types of chondroitin hydro-lyases through HPLC and conducting quantitative determination is shown in Table 8. Further, the degree of sulfation (number of sulfuric acid groups per molecule of GalNAc) of the chondroitin sulfate obtained therefrom was shown in Table 9.

From Table 8, it was found that the chondroitin sulfate of the invention had an intermediate structure between the whale-origin chondroitin sulfate and the shark-origin chondroitin sulfate. Accordingly, it is presumed that the chondroitin sulfate of the invention can be applied to a relatively wide range.

**Table 8**

| Unsaturated disaccharides constituting chondroitin sulfate | | | | |
|---|---|---|---|---|
| | ΔDi-0S | ΔDi-6S | ΔDi-4S | ΔDi-di4,6S |
| ChS-S (%) | 11.0 | 52.8 | 28.4 | 7.8 |
| ChS-A (%) | 3.6 | 25.3 | 70.2 | 0.6 |
| ChS-C (%) | 7.7 | 71.2 | 13.2 | 7.6 |
| ΔDi-0S : non-sulfated GalNAc | | | | |
| ΔDi-6S : C6-monosulfated GalNAc | | | | |
| ΔDi-6S : C4-monosulfated GalNAc | | | | |
| ΔDi-di4,6S : C4,C6-disulfated GalNAc | | | | |

**Table 9**

| Degree of sulfation of each chondroitin sulfate | | | |
|---|---|---|---|
| | ChS-S | ChS-A | ChS-C |
| Degree of sulfation | 0.968 | 0.973 | 1.002 |

### Industrial Applicability

The chondroitin sulfate of the invention has an intermediate structure between the hitherto known whale-origin chondroitin sulfate and shark-origin chondroitin sulfate. Accordingly, there is a high possibility that it can be applied to a relatively wide range. Like the ordinary products, it can be used in drugs such as an anti-inflammatory agent and the like, further in cosmetics or eye lotion as a humectant and in food additives such as a gelling agent, a jellying agent. Moreover, since the average molecular weight is higher than that of ordinary ones, it is considered that a physiological viscosity is higher than that of ordinary ones. Thus, by blending with combined drugs, an effect of prolonging a local res idence time of the drugs can be expected. In addition, there is a recent report on the chondroitin sulfate, stating - that it expedites stabilization of corneal collagen filaments and is effective for maintaining the function of the eye tissue. Therefore, the application as a highly-functional skin substitute can also be expected by blending with collagens such as these extracted from a salmon skin, a cattle skin. Besides, since it exhibits not only the physiological and pharmaceutical activities but also characteristics as a polymeric electrolyte, the industrial application can be expected too.

## Claims

1. A chondroitin sulfate comprising a disaccharide unit containing a non-sulfated N-acetyl-D-galactosamine: (11.0 ± 3.3)%, a disaccharide unit containing a C6-monosulfated N-acetyl-D-galactosamine: (52.8 ± 15.8)%, a disaccharide unit containing a C4-sulfated N-acetyl-D-galactosamine: (28.4 ± 8.5)% and a disaccharide unit containing a C4,C6-disulfated N-acetyl-D-galactosamine: (7.8 ± 2.3)%.

2. The chondroitin sulfate according to claim 1, which has a structure that a disaccharide unit containing a non-sulfated N-acetyl-D-galactosamine, a disaccharide unit containing a C6-monosulfated N-acetyl-D-galactosamine, a disaccharide unit containing a C4-monosulfated N-acetyl-D-galactosamine and a disaccharide unit containing a C4,C6-disulfated N-acetyl-D-galactosamine are randomly arranged.

3. The chondroitin sulfate according to claim 1, which is obtained from salmon nasal cartilage.

4. A process for producing the chondroitin sulfate according to claim 1, which comprises grinding salmon nasal cartilage at a low temperature, defatting the ground matter, then treating it with an alkali and a pronase, centrifuging the resulting liquid digested matter, and then precipitating it with ethanol.

5. The process for producing the chondroitin sulfate according to claim 4, which further comprises treating the resulting precipitate with a cation exchange resin.

6. An anti-inflammatory agent containing the chondroitin sulfate according to claim 1.

7. A humectant containing the chondroitin sulfate according to claim 1.

## Patentansprüche

1. Chondroitinsulfat, das eine Disaccharid-Einheit, welche ein nicht-sulfiertes N-Acetyl-D-galactosamin enthält: (11,0 ± 3,3 %), eine Disaccharid-Einheit, welche ein C6-monosulfiertes N-Acetyl-D-Galactosamin enthält: (52,8 ± 15,8 %), eine Disaccharid-Einheit, welche ein C4-sulfiertes N-Acetyl-D-Galactosamin enthält: (28,4 ± 8,5 %) und eine Disaccharid-Einheit, welche ein C4,C6-disulfiertes N-Acetyl-D-Galactosamin enthält: (7,8 ± 2,3 %), umfasst.

2. Chondroitinsulfat nach Anspruch 1, welches eine Struktur aufweist, bei der eine Disaccharid-Einheit, welche ein nicht-sulfiertes N-Acetyl-D-Galactosamin enthält, eine Disaccharid-Einheit, welche ein C6-monosulfiertes N-Acetyl-D-Galactosamin enthält, eine Disaccharid-Einheit, welche ein C4-monosulfiertes N-Acetyl-D-Galactosamin enthält und eine Disaccharid-Einheit, welche ein C4,C6-disulfiertes N-Acetyl-D-Galactosamin enthält, willkührlich angeordnet sind.

3. Chondroitinsulfat nach Anspruch 1, welches aus Lachs-Nasenknorpel erhalten wird.

4. Verfahren zum Herstellen eines Chondroitinsulfats nach Anspruch 1, welches das Mahlen von Lachs-Nasenknorpel bei einer niedrigen Temperatur, das Entfetten des gemahlenen Materials, dann dessen Behandeln mit einem Alkali und einer Pronase, das Zentrifugieren des resultierenden flüssigverdauten Materials und dann sein Ausfällen mit Ethanol umfasst.

5. Verfahren zum Herstellen des Chondroitinsulfats nach Anspruch 4, welches außerdem das Behandeln des resultierenden Präzipitats mit einem Kationenaustauscherharz umfasst.

6. Entzündungshemmendes Mittel, das das Chondroitinsulfat nach Anspruch 1 enthält.

7. Feuchthaltemittel, das das Chondroitinsulfat nach Anspruch 1 enthält.

## Revendications

1. Sulfate de chondroïtine comprenant un motif disaccharide contenant une N-acétyl-D-galactosamine non sulfatée : (11,0 ± 3,3) %, un motif disaccharide contenant une N-acétyl-D-galactosamine monosulfatée en C6 : (52,8 ± 15,8) %, un motif disaccharide contenant une N-acétyl-D-galactosamine sulfatée en C4 : (28,4 ± 8,5) % et un motif disaccharide contenant une N-acétyl-D-galactosamine disulfatée en C4,C6 : (7,8 ± 2,3) %.

2. Sulfate de chondroïtine selon la revendication 1, qui a une structure dans laquelle un motif disaccharide contenant une N-acétyl-D-galactosamine non sulfatée, un motif disaccharide contenant une N-acétyl-D-galactosamine monosulfatée en C6, un motif disaccharide contenant une N-acétyl-D-galactosamine monosulfatée en C4 et un motif disaccharide contenant une N-acétyl-D-galactosamine disulfatée en C4,C6 sont disposés de façon aléatoire.

3. Sulfate de chondroïtine selon la revendication 1, qui est obtenu à partir de cartilage nasal de saumon.

4. 'Procédé pour préparer le sulfate de chondroitine selon la revendication 1, qui comprend le broyage de cartilage nasal de saumon à une faible température, le dégraissage de la matière broyée, puis le traitement de celle-ci avec un alcali et une pronase, la centrifugation de la matière digérée liquide obtenue puis la précipitation de celle-ci avec de l'éthanol.

5. Procédé pour produire le sulfate de chondroïtine selon la revendication 4, qui comprend en outre le traitement du précipité obtenu avec une résine échangeuse de cations.

6. Agent anti-inflammatoire contenant le sulfate de chondroïtine selon la revendication 1.

7. Humectant contenant le sulfate de chondroïtine selon la revendication 1.
